(19) Europäisches Patentamt
European Patent Office
Office européen des brevets

(11) Numéro de publication: **0 360 677 B1**

(12) **FASCICULE DE BREVET EUROPEEN**

(45) Date de publication de fascicule du brevet: **06.04.94**

(51) Int. Cl.[5]: **C12Q 1/68**

(21) Numéro de dépôt: **89402549.3**

(22) Date de dépôt: **18.09.89**

# (54) Procédé et installation d'identification des bases d'un ADN.

(30) Priorité: **20.09.88 FR 8812285**

(43) Date de publication de la demande:
**28.03.90 Bulletin 90/13**

(45) Mention de la délivrance du brevet:
**06.04.94 Bulletin 94/14**

(84) Etats contractants désignés:
**CH DE ES FR GB LI NL SE**

(56) Documents cités:
**WO-A-85/05642**
**WO-A-89/12694**
**DE-A- 3 312 929**
**FR-A- 2 558 262**
**US-A- 4 022 876**

**PATENT ABSTRACTS OF JAPAN, vol. 10, no. 111 (P-451)[2168], 25 avril 1986&NUM;**

**TETRAHEDRON LETTERS, vol. 26, no. 40, 1985; pp. 4915-4918&NUM;**

(73) Titulaire: **COMMISSARIAT A L'ENERGIE ATOMIOUE**
**31/33, rue de la Fédération**
**F-75015 Paris Cédex 15(FR)**

(72) Inventeur: **De Cosnac, Bertrand**
**11 Bis, avenue Mac Mahon**
**F-75017 Paris(FR)**
Inventeur: **Grimm, Bertrand**
**Ecole Centrale**
**F-92295 Chatenay Malabry Cédex(FR)**
Inventeur: **Haan, Serge**
**18 Domaine de la Butte à la Reine**
**F-91120 Palaiseau(FR)**

(74) Mandataire: **Des Termes, Monique et al**
**Société Brevatome**
**25, rue de Ponthieu**
**F-75008 Paris (FR)**

**EP 0 360 677 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention se rapporte à un procédé et une installation d'identification des motifs d'une molécule organique complexe et en particulier d'une molécule biologique complexe telle qu'un acide nucléique , une protéine, un peptide, un polymère. Elle s'applique dans les domaines du génie génétique, de la biologie, de la pharmacologie, tant au stade de l'expérimentation, de la recherche fondamentale qu'industrielle.

Le procédé de l'invention s'applique de façon générale à toute molécule constituée d'une succession d'acides aminés, de nucléotides, de groupes d'atomes et, plus spécialement, aux acides nucléiques, tels que l'ADN (acide désoxyribonucléique) et l'ARN (acide ribonucléique).

L'identification des séquences ou de l'ordre des nucléotides de l'ADN ou l'ARN est connue sous le nom de séquençage.

Le séquençage des acides nucléiques a pris une réelle importance dans le domaine de la biologie moléculaire et de la biotechnologie. En particulier, le séquencage de l'ADN permet l'analyse des génomes animaux, végétaux et viraux dans des gènes particuliers. Puisque la fonction d'une molécule biologique est déterminée par sa structure, la définition de la structure d'un gène est cruciale pour la manipulation éventuelle de cette unité de base d'information.

On rappelle qu'un nucléotide est formé par une base, un sucre et un phosphate.

Pour les acides nucléiques, les bases sont des bases azotées possédant une structure hétérocyclique du type purique ou pyrimidique ; elles sont au nombre de 4. Le sucre est le ribose pour l'ARN et le désoxyribose pour l'ADN et le phosphate l'acide phosphorique.

Généralement, les nucléotides des acides nucléiques utilisés sont triphosphatés car c'est ce type de molécules qui fournit l'énergie nécessaire au fonctionnement des enzymes dans de nombreuses réactions biochimiques, en particulier celles intervenant dans la polymérisation des ADN.

L'ARN et l'ADN présentent trois bases communes : l'adénine (A) et la guanine (G) qui sont des purines et la cytosine (C) qui est une pyrimidine. L'autre base pyrimidique est la thymine (T) pour l'ADN et l'uracile (U) pour l'ARN.

Pour l'ADN, les quatre types de nucléotides concernés sont le désoxy adénosine triphosphate ou dATP, le désoxy guanine triphosphate ou dGTP, le désoxy cytosine triphosphate ou dCTP et le désoxy thymine triphosphate ou dTTP. On les note ci-après dXTP avec X = A, G, C ou T.

L'ADN contient l'information génétique complète nécessaire à la fabrication des protéines, éléments constitutifs de tout organisme vivant. En particulier, chaque succession de trois bases est le code, par l'intermédiaire des ARN messagers, d'un acide aminé qui entre dans la composition d'une protéine. Le séquençage a pour but d'obtenir l'ordre des bases constituant un brin d'ADN ou d'ARN.

Les méthodes de séquençage de l'ADN comportent essentiellement 5 étapes qui sont (a) la fragmentation du génome, (b) les réactions chimiques de séquençage pour former des chaînes de nucléotides, (c) le marquage des chaînes de nucléotides, (d) la séparation des chaînes marquées et (e) la détection.

Etape a

Un génome ou ensemble des secteurs de l'ADN est constitué par un grand nombre de paires de bases (13 x $10^6$ pour la levure et 3 x $10^9$ pour l'homme). Le génome, du fait de sa longueur, ne peut être directement soumis aux réactions de séquençage. L'ADN est scindé en fragments d'environ 1000 à 30000 bases qui sont ensuite multipliés par clonage. Par exemple, cette fragmentation peut être effectuée par l'utilisation d'enzymes de restriction qui coupent l'ADN au niveau de certaines séquences ou par l'utilisation des ultra-sons, comme exposé dans l'article "Current Approaches to DNA Sequencing" de J.C. Moores Analytic Biochemistry 163, 1-8 (1987).

Etape b

Elle consiste à appliquer aux chaînes obtenues en (a) quatre séries de réactions chimiques spécifiques des quatre bases de l'ADN. Ces réactions conduisent à l'obtention de quatre familles de chaînes d'ADN dont l'une des extrémités est communément fixée et l'autre est constituée d'une base spécifique de l'une des quatre séries de réaction.

Deux méthodes sont communément utilisées pour obtenir les 4 familles de chaînes d'ADN ci-dessus. L'une, dévelopée par Maxam et Gilbert (Maxam A.M. et Gilbert W., Proc. Natl. Acad. Sci., U.S.A., vol. 74, N° 2, pp 560-564 de 1977, "A new method for sequencing DNA") fait appel à une procédure de dégradation chimique des chaînes et l'autre, introduite par Sanger (Sanger F. et al., Proc. Natl. Acad. Sci. USA 74, pp. 5453-5467 de Décembre 1977 "DNA sequencing with chain-terminating inhibitors") est une

méthode enzymatique et emploie un procédé de terminaison de chaînes didésoxy.

Dans la méthode de Sanger, chacune des quatre réactions de séquençage met en jeu un grand nombre d'exemplaires d'ADN monocaténaire à séquencer ; un amorceur (ou en terminologie anglo-saxonne primer) qui est un acide-nucléique complémentaire de l'une des extrémités de l'ADN à séquencer ; les quatre nucléotides dXTP ; une enzyme qui permet la fabrication du brin complémentaire de l'ADN à séquencer, à partir des nucléotides et en commençant par l'amorceur et enfin un seul des quatre di-désoxy nucléotides appelé nucléotide terminal et noté ddXTP. Ce ddXTP est un nucléotide auquel il manque le groupement OH du carbone 3′ du désoxyribose.

L'amorceur se lie à l'ADN à séquencer par complémentarité puis l'enzyme synthétise le brin complémentaire jusqu'à ce qu'elle incorpore le ddXTP arrêtant l'élongation de la chaîne. L'incorporation du nucléotide terminal peut se produire n'importe quand au cours de la synthèse ce qui se traduit, à la fin de l'opération de séquençage spécifique d'une base X donnée, par la présence d'une famille d'ADN représentant toutes les longueurs possibles depuis l'amorceur jusqu'à environ 400 nucléotides.

Appliquée en parallèle pour les quatre ddXTP, la série de quatre réactions conduit à un ensemble de quatre familles de chaînes d'ADN ; les chaînes de chaque famille étant représentatives d'une base X donnée sont ensuite déposées sur un gel d'électrophorèse.

Dans la méthode de Maxam et Gilbert, des chaînes d'ADN de dimensions adaptées et analogues à celles issues du procédé de Sanger sont produites par un traitement chimique d'ADN purifié. Cette opération permet de séquencer un ADN mono- ou bicaténaire marqué avec un phosphate radioactif à l'une de ses extrémités.

Pour chacune des quatre séries de réaction de séquençage, l'ADN est soumis à un jeu de deux réactions chimiques. la première réaction chimique modifie et retire la base de son sucre, la seconde coupe l'ADN en retirant le sucre ainsi exposé. On obtient ainsi des chaînes commençant à un endroit fixé et se terminant par une base connue. Les conditions de réaction sont ajustées de façon à produire des chaînes de toutes les longueurs possibles comportant typiquement de 1 à 400 nucléotides.

<u>Etape c</u>

Les chaînes obtenues en (b) sont marquées préalablement, ou au cours des réactions, avec des marqueurs radioactifs. Les marqueurs radioactifs utilisés sont le $^{32}P$ ou le $^{35}S$, le tritium, le $^{14}C$ qui sont des émetteurs de rayonnements $\beta$-.

Dans le cadre de la méthode de Sanger, il est possible de marquer l'amorceur, les dXTP ou les ddXTP.

<u>Etape d</u>

Les chaînes d'ADN marquées sont alors séparées suivant leur longueur par électrophorèse, les plus petites migrant le plus vite et les plus grandes le plus lentement. Le gel d'électrophorèse qui est en général un gel de polyacrylamide permet de différencier les molécules de longueur N et N+1 avec N variant de 1 à 400 environ.

L'électrophorèse dure de 4 à 12 heures suivant la longueur du gel qui est ensuite séché.

<u>Etape e</u>

Le gel est ensuite placé contre un film sensible au rayonnement béta. Les marqueurs radioactifs incorporés dans les chaînes d'ADN sont utilisés pour développer une image auto-radiographique de chaque tracé électrophorétique. L'auto-radiographie est développée après 12 heures ou plus d'exposition au rayonnement.

La position relative des bandes qui apparaissent sur l'image auto-radiographique, au sein des quatre colonnes correspondant aux quatre familles de réactions, permet la détermination de la séquence des bases de l'ADN de départ.

Après la lecture, l'examen et l'interprétation de l'auto-radiographie, la séquence est entrée dans la mémoire d'un ordinateur pour une analyse et un assemblage ultérieurs.

Ces deux méthodes de séquençage d'ADN précédemment décrites sont largement utilisées et comportent chacune plusieurs variantes. Bien que très performantes et efficaces, ces méthodes demandent une mise en oeuvre très complexe qui comporte de sérieux inconvénients. C'est en réalité un très gros travail, délicat, qui doit être extrêmement bien standardisé pour que la source de connaissances et la comparaison des migrations électrophorétiques soient fiables.

Par ailleurs, la lecture des électrophorèses des chaînes radioactives se fait après auto-radiographie. Là aussi, une parfaite reproductibilité est de rigueur tant dans le développement de ces auto-radiogrammes que dans leur lecture.

En effet, l'image sur film d'une bande de gel radioactif est plus large que la bande elle-même et la comparaison des positions des bandes entre les quatre pistes différentes de l'électrophorèse (qui peuvent induire un comportement non uniforme quant aux mobilités des chaînes) peut limiter la résolution effectivement observée sur l'auto-radiogramme et donc réduire la longueur de la séquence qui peut être lue à partir des gels. Typiquement, de 200 à 400 bases peuvent être lues à partir d'un simple jeu de tracés.

De plus, les irrégularités survenant d'un tracé à un autre peuvent rendre difficile la lecture automatique des auto-radiogrammes par des techniques d'imagerie existantes (par exemple densitomètres à balayages) car l'oeil humain peut actuellement mieux discriminer ces irrégularités que ne le font les ordinateurs.

La nécessité d'un soin intensif quant à l'analyse "manuelle" des auto-radiogrammes demande donc du temps et est sujette à des erreurs.

Par ailleurs, l'utilisation de marqueurs radioactifs présente de sérieux inconvénients quant à la manipulation (système de protection complexe des opérateurs et décontamination périodique des installations), au stockage des produits durant l'analyse et au stockage des déchets en fin d'analyse.

Enfin, il n'est pas possible d'observer les bandes au sein du gel pendant l'électrophorèse afin de contrôler l'opération en cours, mais il faut nècessairement terminer l'électrophorèse à un moment déterminé et attendre le développement de l'auto-radiogramme avant de pouvoir lire la séquence.

Aussi, plusieurs procédés ont déjà envisagé la détection en temps réel des chaînes d'ADN pendant leur migration au cours de l'électrophorèse.

Les uns s'intéressent par exemple à la détection du rayonnement $\beta$- émis par les chaînes marquées au $^{32}P$, les autres s'intéressent à la détection de l'émission de fluorescence induite par un faisceau laser.

Dans le premier cas, la détection en ligne (ou temps réel) des bandes radioactives au cours de l'électrophorèse peut permettre une certaine simplification, par une certaine réduction du nombre des opérations à effectuer, pour l'ensemble du procédé. Cependant, il faut tenir compte de la courte période de demi-vie du phosphore 32 et par conséquent de l'instabilité des réactifs de marquage radioactif. En outre, comme dans les méthodes plus traditionnelles de Sanger ou Maxam et Gilbert, il demeure que des problèmes sont posés par l'élimination et la manipulation des produits radioactifs.

En outre, le temps de passage des chaînes devant le détecteur étant très réduit, cela implique une plus faible efficacité de collection des rayonnements, ce qui peut entraîner des problèmes de sensibilité et de résolution.

La détection de l'émission de fluorescence induite par un faisceau laser a été proposée par Ansorge (voir DE-A-3 618 605 et l'article Journal of Biochemical and Biophysical Methods. 13(1986), pp 315-323, "A non-radioactif automated method for DNA sequence determination"). Cette méthode utilise un seul fluorophore pour marquer l'amorceur (ou primer) dans le cadre de la méthode de Sanger.

Dans ce cas, les quatre séries de réactions de séquençage conduisent, comme décrit précédemment, à quatre familles de chaînes d'ADN déposées sur quatre pistes parallèles d'un gel d'électrophorèse, qui est traversé entièrement, suivant sa largeur, par un faisceau laser. Au sein du gel, les chaînes d'ADN marquées sont excitées les unes après les autres, dans l'ordre de la séquence, lorsqu'elles passent dans le faisceau laser, au cours de l'électrophorèse.

Pour chaque piste d'électrophorèse associée à une famille de réactions donnée, la lumière de fluorescence est collectée par une optique adaptée et conduite, après filtrage, à un détecteur de type photomultiplicateur.

Un avantage offert par cette technique est l'absence de toute partie mobile au sein de l'appareil de détection grâce aux détecteurs fixes qui permettent une détection continue sur les quatre pistes d'électrophorèse.

Toutefois, l'utilisation d'un seul fluorophore conduit inévitablement à l'utilisation de quatre pistes en parallèle, sur le gel d'électrophorèse, pour l'étude d'une seule séquence, ce qui limite les possibilités d'évolution sensible et de simplification ultérieures. Il en est de même lors de la détection en temps réel des chaînes d'ADN marquées par du phosphore 32.

Ces systèmes de détection en temps réel nécessitent aussi une grande fiabilité quant à la comparaison des mobilités électrophorétiques d'une piste à l'autre. Or, en pratique, plusieurs difficultés peuvent être rencontrées ; en particulier, la formation de gradient thermique et la présence d'impuretés au sein du gel peuvent induire des distorsions locales des chaînes au sein du gel, et par suite compromettre directement l'attribution des bases dans la séquence cherchée.

D'autres procédés de détection en temps réel proposent l'utilisation de plusieurs fluorophores afin d'identifier individuellement chaque famille de chaînes. Ce type de procédé présente l'avantage, dans son

concept, d'offrir la possibilité de séparer des chaînes d'ADN issues des réactions de séquençage, sur une seule et même piste d'électrophorèse. Ces systèmes utilisent une série de quatre fluorophores, ce qui semble répondre aux divers problèmes soulignés précédemment. Toutefois, la mise en oeuvre spécifique de chacun de ces systèmes ne rend ces approches que partiellement réussies.

Un premier système a été proposé par L.M. Smith et al. (voir FR-A-2 558 262), dans le cadre des réactions de séquençage de Sanger. Dans cette technique, les réactions de séquençage doivent s'effectuer dans quatre tubes séparés, chacun comprenant un marqueur fluorescent différent lié au "primer". L'appareil de détection décrit par Smith et al. utilise une série de filtres interférentiels à bande étroite afin de sélectionner les longueurs d'onde spécifiques d'émission de chacun des fluorophores.

A partir de ce procédé relativement simple, le type de système qui en résulte comporte au demeurant certaines imperfections. En particulier, de sérieux problèmes sont engendrés par l'utilisation de molécules fluorescentes de charges et de tailles très différentes.

Cette situation se traduit par l'existence de perturbations au cours de l'électrophorèse dues aux différences de mobilités électrophorétiques des diverses chaînes marquées différemment. En effet, malgré l'élaboration conjointe d'un logiciel lourd et assez complexe de traitement et de correction des données, ces phénomènes peuvent entraîner certains recouvrements voire des inversions de chaînes au cours de l'électrophorèse, empêchant ou même faussant l'identification de certaines bases le long de la séquence.

Par ailleurs, cette technique requiert obligatoirement l'utilisation de quatre "primers" spécifiques marqués. Ceci entraîne, dans les cas où d'autres vecteurs sont employés, la nécessité d'entreprendre des processus longs et complexes de synthèse et de purification de quatre nouveaux "primers" fluorescents.

Un second système proposé par J.M. Prober et al. (voir EP-A-0 252 683) repose, toujours dans le cadre des réactions Sanger, sur l'utilisation de quatre fluorophores liés aux quatre ddXTP. Ce marquage différent des ddXTP procure un certain nombre d'avantages. En particulier, les quatre réactions de séquençage peuvent s'effectuer dans un même tube, un amorceur unique non marqué peut alors être utilisé et les produits de réaction peuvent être séparés au sein d'une même colonne d'électrophorèse.

Cependant, le fait de greffer au ddXTP des marqueurs extrinsèques, c'est-à-dire les fluorophores, qui sont des molécules assez complexes et très volumineuses, peut entraîner des décalages imprévisibles dans les mobilités de certaines chaînes au cours de l'électrophorèse et surtout restreindre fortement le choix d'enzymes de complémentarité ; ceci élimine la possibilité d'utilisation des enzymes courantes comme amorceur, comme par exemple le fragment de Klenow d'ADN polymérase. Les fluorophores utilisés sont généralement les dérivés de la fluorescéine.

Par ailleurs, il est connu du document WO 89/12 694 opposable selon l'article 54(3) CBE, l'utilisation d'isotopes stables du carbone, du soufre, du chlore et du brome pour le marquage des chaînes de nucléotides de tailles différentes, la séparation et l'ionisation de ces chaînes par électrophorèse, ainsi que la détection des ions formés par spectrométrie de masse.

L'invention a donc pour objet un procédé d'identification des séquences d'un ADN permettant de remédier à la plupart des inconvénients donnés précédemment. Des problèmes similaires apparaissant dans le séquençage de tout autre acide nucléique et, en particulier, dans les ARN, l'invention s'applique aussi à toute autre molécule complexe à structure répétitive (protéine ou peptide).

L'invention a pour objet un procédé d'identification de la séquence des bases d'un acide nucléique, caractérisé en ce ou'il comprend les étapes suivantes :

A) - production de fragments de l'acide nucléique,

B) - réactions chimiques spécifiques des bases pour former des familles de chaînes de taille différente, chaque famille comportant à une extrémité une base spécifique,

C) - marquage spécifique desdites familles de chaînes et marquage de chaque nucléotide de chaque chaîne à l'aide d'isotopes stables ou de petites molécules stables directement greffables sur les nucléotides desdites chaînes.

D) - introduction une à une des chaînes marquées dans des moyens d'atomisation et d'ionisation,

E) - atomisation et ionisation de chaque chaîne marquée à l'aide desdits moyens,

F) - sélection et détection en temps réel des ions formés au fur et à mesure de leur formation, par spectrométrie de masse, et

G) -traitement des signaux issus de la spectrométrie de masse, pour déterminer la longueur de chaque chaîne en comptant le nombre de nucléotides marqués de chaque ion détecté et pour identifier le marquage spécifique de chaque famille de chaînes.

Le fonctionnement en temps réel est lié au fait que, dans l'invention, les ions sont détectés au fur et à mesure de leur formation.

L'ionisation des chaînes marquées puis la détection de ces chaînes par un spectromètre ou analyseur de masse facilite considérablement la détection des chaînes marquées et donc l'identification des bases de

l'acide nucléique.

Le procédé selon l'invention s'applique parfaitement bien au séquençage de l'ADN.

Ce procédé de séquençage fonctionne en continu et ne comporte pas d'étape de radiographie ni d'électrophorèse selon l'art antérieur. En outre, les chaînes de nucléotides marquées sont examinées et analysées une par une et non globalement selon l'art antérieur.

Selon l'invention, on effectue un marquage de tous les nucléotides de chaque chaîne ; la détection des marqueurs de chaque nucléotide donne l'information de la longueur de ces chaînes alors que la détection du marquage spécifique donne l'information de la famille de chaînes.

Enfin, l'ionisation et la spectrométrie de masse permettent l'utilisation de marqueurs liés de manière intrinsèque ou extrinsèque aux différents nucléotides, ce qui autorise l'application de ce procédé aux diverses méthodes chimiques de séquençage les plus répandues, et en particulier, celle de Sanger et de ses variantes.

Le marquage spécifique de chaque famille de chaînes et/ou le marquage de tous les nucléotides de chaque chaîne sont effectués avec des isotopes ou des petites molécules stables.

Par petites molécules, il faut comprendre des composés chimiques pouvant être liés aux nucléotides et qui comportent au moins un élément marqueur identifiable par spectrométrie de masse, tel que $CF_2$, $Si(CH_3)_3$, en utilisant par exemple une technique décrite dans l'article Tetrahedron, vol. 26, N° 40, pp. 4915-4918 (1985), C.D. Pein, D. Cech.

Le procédé selon l'invention, grâce à l'utilisation de trois, quatre ou plusieurs isotopes ou petites molécules stables, c'est-à-dire non radioactifs, donc non dangereux pour les opérateurs, facilite considérablement les interventions humaines dans le procédé d'identification.

L'invention a aussi pour objet une installation pour l'identification des bases d'un acide nucléique comportant :

- une source de familles de chaînes de bases de longueur différente, chaque famille de bases étant spécifiquement marquée et les nucléotides de chaque chaîne étant marqués, ces marquages étant faits avec des isotopes stables ou de petites molécules stables directement greffées sur les nucléotides desdites chaînes,
- des moyens pour introduire une à une dans des moyens d'atomisation les chaînes marquées,
- des moyens d'atomisation de chaque chaîne marquée,
- des moyens d'ionisation des chaînes individualisées et atomisées,
- un spectromètre de masse équipé de plusieurs détecteurs pour sélectionner et détecter les ions formés au fur et à mesure de leur formation, et
- des moyens de traitement des signaux fournis par les détecteurs, pour déterminer la longueur de chaque chaîne en comptant le nombre de nucléotides marqués de chaque ion détecté et pour identifier le marquage spécifique de chaque famille de chaînes.

Comme on l'a dit précédemment, l'invention s'applique à toute autre molécule complexe que l'ADN et l'ARN à structure répétitive. Aussi, l'invention a encore pour objet un procédé d'identification des motifs d'une molécule organique complexe ou d'un fragment de cette molécule comportant les étapes suivantes :

- marquage spécifique desdits motifs par des isotopes stables ou petites molécules stables directement greffables sur lesdits motifs,
- introduction un à un des motifs marqués dans des moyens d'atomisation et d'ionisation,
- atomisation et ionisation de chaque motif marqué à l'aide desdits moyens,
- détection en temps réel des ions formés au fur et à mesure de leur formation par spectrométrie de masse, et
- traitement des signaux issus de la spectrométrie de masse pour déterminer la longueur de la molécule complexe ou du fragment de cette molécule en comptant le nombre de motifs marqués et pour identifier leur marquage spécifique.

D'autres caractéristiques et avantages de l'invention ressortiront de la description qui suit, donnée à titre illustratif et non limitatif aux dessins annexés, dans lesquels :

- la figure 1 représente un schéma d'ensemble des étapes de séquençage automatisé d'ADN selon l'invention,
- la figure 2 représente un schéma d'ensemble d'une installation automatisée pour le séquençage d'ADN conforme à l'invention,
- la figure 3 représente le type de données produites par un séquençage d'ADN selon l'invention.

La description qui va suivre se réfère au séquençage de l'ADN, bien que comme on l'a dit précédemment, l'invention s'applique à tout acide nucléique (ARN par exemple) et même à toute molécule organique complexe (protéine, peptide).

La première étape du procédé, en référence à la figure 1, consiste à fragmenter un génome d'ADN (étape a), conformément à l'art antérieur cité. Cette étape de fragmentation porte la référence 2 sur la figure 1.

Les chaînes obtenues sont alors soumises aux réactions de séquençage (étape b) selon les méthodes de Sanger, si ce n'est que l'échelle peut être augmentée en cas de besoin pour fournir une intensité de signal adéquate pour la détection de chaque famille de chaînes. Aucun nucléotide radio marqué n'a besoin d'être inclu dans les réactions de séquençage. Les réactions de sequençage sont symbolisées par le bloc 4 sur la figure 1.

Selon l'invention, tous les nucléotides incorporés selon Sanger au cours de la synthèse des chaînes nucléotidiques complémentaires du fragment d'ADN à séquencer sont marqués ou bien comportent une caractéristique identifiable par spectromérie de masse. En outre, un marquage spécifique de chaque famille de chaînes issue des réactions de séquençage doit être effectué.

Le marquage des chaînes nucléotidiques, symbolisé par le bloc 6, peut être réalisé en utilisant un isotope ou une combinaison d'isotopes stables, constituants éventuels de petites molécules stables, afin de marquer spécifiquement chaque nucléotide de chaque chaîne ainsi que l'extrémité de ces chaînes en fonction de la base terminale de ces chaînes.

Le marquage spécifique de la famille de chaîne peut être réalisé sur l'amorceur, comme décrit dans le document FR-A-2 558 262, ou sur le didésoxy nucléotide, comme décrit dans le document EP-A-0 252 683, mais en utilisant des isotopes ou petites molécules stables.

Grâce à ce double système de marquage, chaque chaîne d'ADN issue des réactions chimiques de séquençage comporte une double information : 1) la détection du nombre de marqueurs et donc du nombre de nucléotides constituant chaque chaîne, qui donne l'information taille de la chaîne (comme le faisait antérieurement l'électrophorèse -étape d-), 2) la détection du marqueur stable spécifique de la famille qui donne le type de nucléotide, et donc de base, existant à l'une des extrémités de la chaîne (comme le faisait antérieurement les fluorophores ou les isotopes radioactifs -étape c-).

Le marquage isotopique selon l'invention des chaînes d'ADN peut être réalisé au niveau de l'un des trois composés chimiques constituant les nucléotides, c'est-à-dire la base, le sucre ou le groupement triphosphate ou éventuellement sur un groupement alpha-thiophosphaté dans lequel l'atome d'oxygène lié au premier phosphore est remplacé par un atome de soufre.

Le marquage des chaînes peut être réalisé de façon intrinsèque sur le carbone, l'hydrogène, l'azote, l'oxygène ou même le soufre entrant dans la constitution des trois composés chimiques d'un nucléotide. L'utilisation d'isotopes naturels stables, selon l'invention, offre un choix plus important que les isotopes radioactifs généralement utilisés ($^{32}P$, $^{35}S$, $^{14}C$, $^{3}H$) pouvant entrer dans la constitution des différents composés nucléotidiques.

Une liste de ces isotopes stables, incluant leur abondance naturelle relative, est donnée dans le tableau 1.

Tableau 1

| Isotopes naturels stables pour marquage intrinsèque des composés nucléotiques | |
|---|---|
| Isotopes | Abondance relative (%) |
| D | O,015 |
| $^{13}C$ | 1,10 |
| $^{15}N$ | 0,37 |
| $^{17}O$ | 0,038 |
| $^{18}O$ | 0,20 |
| $^{32}S$ | 95,02 |
| $^{33}S$ | 0,75 |
| $^{34}S$ | 4,21 |
| $^{36}S$ | 0,02 |

Du point de vue chimique, les réactions pour incorporer un isotope stable ou un isotope radioactif sont les mêmes.

Les divers procédés de marquage de molécules biologiques par des isotopes stables existent et sont largement répandus (voir à cet effet l'article "The preparation of purines, pyrimidines, nucléotides and related compounds labelled with $^{17}O$ and other stable isotopes", E. Petreanu, J.S. Cohen and D. Samuel,

Proceedings of the 2nd International Conference on methods of preparing and storing labelled compounds, pp. 489-497). Différentes molécules marquées, en particulier les nucléotides peuvent être produites par voie de synthèse ou par voie enzymatique. En particulier, de nombreux composés nucléotidiques marqués avec des isotopes stables sont disponibles sur le marché pour des applications en biologie et en pharmacologie différentes de celles de l'invention (voir par exemple l'article Dehalogenation reactions in fast atom bombardment mass-spectrometry, Anal. Chem. 1984.56, 1975.77).

Des exemples de nucléotides marqués à l'aide d'isotopes naturels sont donnés dans les annexes I et II.

Ces exemples ont uniquement pour but d'illustrer des moyens de marquage possibles utilisables dans l'invention.

Comme données dans ces annexes I et II, le marquage peut être effectué sur les formes désoxytriphosphate (annexe I) ainsi que sur leurs analogues alpha-thiotriphosphate (annexe II) pour le marquage de chaque nucléotide et peut être effectué sur les formes didésoxytriphosphate (annexe I) ainsi que sur leurs analogues alpha-thiotriphosphate (annexe II) pour le marquage spécifique de la famille.

Comme autre nucléotide marqué intrinsèquement utilisable pour l'information de taille des chaînes issues des réactions de Sanger, on peut citer le 1 ou 3-$^{15}$NdATP, l'α-$^{18}$OdCTP, le 4-$^{18}$OdTTP, le 2-$^{13}$CdATP ou dCTP, le 8-$^{13}$CdATP, le 5′-$^{13}$CdATP, l'α-SdATP, l'α-SdGTP, l'α-SdCTP, l'α-SdTTP où S est l'isotope 32, 34, 33 ou 36, le méthyl $^{13}$CdTTP.

Il est aussi possible, selon l'invention, d'effectuer un marquage extrinsèque des nucléotides de chaque chaîne, des didésoxy nucléotides de ces chaînes ou même des amorceurs. Ce type de marquage comporte l'avantage de pouvoir lier au nucléotide des atomes (sous forme mono ou polyatomique) qui ne sont pas naturellement présents dans ces molécules. Ces marqueurs sont constitués d'éléments ou de petits complexes moléculaires d'éléments alcalins, alcalino-terreux ou halogéniques.

Dans le cadre des réactions de séquençage, le marquage extrinsèque selon l'invention présente moins de risque de dénaturer les propriétés physiques et chimiques des nucléotides, contrairement à l'adjonction selon l'art antérieur de grosses molécules telles que les chromophores ou fluorophores qui ne peuvent être liées que par l'intermédiaire de bras spécifiques relativement complexes. Ainsi, les éléments ou petits complexes moléculaires selon l'invention peuvent être directement greffés au nucléotide en profitant des liaisons covalentes disponibles au sein de la base ou du sucre de chaque nucléotide (voir par exemple l'article d'Anal. Chem. cité en dernier).

Des exemples de marquage extrinsèques selon l'invention sont donnés en annexe III. Cet exemple donne un jeu complet de bases nucléotidiques de l'ARN et ADN marquées extrinséquement. En particulier, X représente un atome de fluor, de chlore, de brome ou d'iode.

Comme nucléotide marqué par des halogènes utilisables dans l'invention, on peut citer le 8-bromo dGTP, le 5-bromo dCTP, le 3′-bromo, chloro ou iodo dTTP, le 5-bromo iodo ou fluoro dTTP, le 3′-FdGTP.

En tenant compte de l'existence de plusieurs isotopes stables possibles des différents marqueurs extrinsèques (éléments halogènes par exemple), les possibilités de marquage isotopiques des nucléotides sont fortement accrues.

Une liste des différents isotopes stables d'éléments halogènes est présentée dans le tableau 2, accompagnés de leur abondance naturelle relative.

Tableau 2

| Isotopes naturels stables des éléments halogènes pour le marquage extrinsèque | |
|---|---|
| Isotopes | Abondance relative (%) |
| $^{19}F$ | 100 |
| $^{35}Cl$ | 75,77 |
| $^{37}Cl$ | 24,23 |
| $^{79}Br$ | 50,69 |
| $^{81}Br$ | 49,31 |
| $^{127}I$ | 100 |

Conformément à l'invention, il est possible d'effectuer des marquages multiples des nucléotides de chaque chaîne issue des réactions de séquençage (information taille), des didésoxy nucléotides et amorceurs (information famille) de ces chaînes, ces marquages étant des marquages intrinsèques et/ou extrinsèques.

En premier lieu, on peut citer des possibilités d'incorporer plusieurs isotopes marqueurs de même type sur un nucléotide donné. Comme nucléotide marqué de ce type, on peut citer par exemple le 1,3-$^{15}N$ dATP, le 1,3-$^{15}N$dCTP, le 2,8-dichloro ou dibromo ou difluoro dATP, le 5,5-dibromo dCTP. Ceci permet d'accroître d'autant la sensibilité obtenue lors de la détection et de l'identification des chaînes nucléotidiques.

En second lieu, plusieurs marqueurs différents peuvent être incorporés au sein d'un même nucléotide afin d'affiner sa signature. De plus, la détection simultanée de plusieurs marqueurs augmente considérablement les combinaisons de marquages possibles.

L'exemple suivant montre que trois types de marqueurs isotopiques stables suffisent à l'identification de quatre nucléotides différents :

| | | | | | |
|---|---|---|---|---|---|
| $^{13}C$ | seul | Nucléotide | de | type | A |
| $^{13}C$ | + $^{17}O$ | " | " | " | G |
| $^{13}C$ | + $^{15}N$ | " | " | " | C |
| $^{15}N$ | + $^{17}O$ | " | " | " | T |

Ainsi, les marquages intrinsèques et extrinsèques des nucléotides à l'aide d'isotopes stables peuvent être combinés et offrent de réelles possibilités multiples et variées.

Cette méthode de marquage s'avère plus souple et plus riche d'évolution que les marquages radioisotopiques ou par fluorophores selon l'art antérieur qui restent forcément plus limités dans leurs possibilités. De plus, la plupart des inconvénients rencontrés avec ces derniers lors de leur manipulation et de leur utilisation sont évités voire complétement éliminés.

Les chaînes d'ADN marquées sont alors placées dans une source d'échantillon 8 appropriée à leur introduction individuelle dans une source d'ions. Cette étape est réalisée grâce à la formation de microgouttelettes contenant au plus une molécule marquée.

Celles-ci sont ensuite introduites successivement une à une dans une source d'ions chargée de l'atomisation et de l'ionisation. Cette étape porte la référence 10 sur la figure 1 et correspond à l'étape D et E.

Les ions formés pour chaque chaîne marquée sont alors sélectionnés puis détectés par spectrométrie de masse, comme indiqué en 12, et les signaux issus de la spectrométrie de masse sont traités, comme indiqué en 14 en vue de reconstituer l'ADN de départ (étape G).

L'installation permettant le séquençage automatisé de l'ADN, conformément à l'invention, est schématisée sur la figure 2. La fragmentation du génome, les réactions chimiques de séquençage et le marquage étant effectués de façon discrète ou de façon indépendante dans le temps, ne sont pas représentés sur la figure 7.

L'installation représentée comporte une source 18 de chaînes marquées d'ADN à l'aide d'isotopes stables selon l'invention, placée sous pression et contenant une cuve 20 destinée à recevoir l'échantillon 22 des chaînes marquées d'ADN diluées à environ $10^{-10}$ mole/l dans une solution tampon.

Un tube capillaire 24 d'environ une dizaine de micron de diamètre plongeant dans l'échantillon 22 assure l'introduction une à une des chaînes d'ADN marquées dans une source d'ions 26. Le cristal piézoélectrique 28 permet le fractionnement homogène la nébulisation en microgouttelettes de quelques micromètres de diamètre de l'affluent du tube 24.

Elles sont alors introduites une à une dans la source à plasma 32 équipée de bobines électromagnétiques 33 reliées à un générateur radiofréquence 34. Dans la zone 32a la plus chaude du plasma, où la température atteint 6727 à 7727 °C (7000 à 8000 °K), les molécules d'ADN sont atomisées et ionisées.

La torche à plasma est la source préférée d'ionisation selon l'invention car elle permet l'atomisation complète des molécules et induit une simplicité maximale dans le spectre de masse obtenu après analyse d'éléments simples.

Les ions formés sont alors pris en charge par l'optique d'entrée 36 d'un spectromètre de masse 38 qui peut être du type à secteur magnétique où un vide différentiel s'installe grâce à des pompes primaires et secondaires 39 ; la pression au niveau de l'optique ionique 36 est de l'ordre de $10^{-4}$ torr ($10^{-2}$Pa) et la pression au niveau des pièces polaires 40 du spectromètre est de l'ordre de $10^{-6}$ torr ($10^{-4}$ Pa).

Le premier rôle du spectromètre de masse 38 est de sélectionner et d'identifier grâce à son optique ionique 36 et ses pièces polaires 40, la présence des masses spécifiques obtenues grâce au traceur isotopique utilisé précédemment. Le spectromètre de masse 38 peut opérer en mode sélectif ce qui consiste à balayer uniquement les masses caractéristiques dues à la présence du marqueur isotopique, afin

de fournir un maximum de sensibilité.

La détection par spectrométrie de masse permet à la fois de détecter les chaînes d'ADN marquées au fur et à mesure de leur sortie de la source de fragments 18, d'identifier, grâce à leurs marqueurs spécifiques, à quelle famille elles correspondent ainsi que de déterminer leur taille grâce au comptage du nombre de nucléotides.

Les molécules ionisées et sélectionnées par le spectromètre de masse sont alors détectées à l'aide d'une série de détecteurs 42 situés à la sortie du spectromètre 38, du type multiplicateur d'électrons et qui sont sensibles à l'impact d'un seul ion. Les ions sélectionnés peuvent donc être détectés individuellement, ce qui favorise l'obtention d'une sensibilité maximale de l'installation totale de séquençage.

Le signal de comptage fourni par chaque détecteur 42 est alors reçu dans un micro-ordinateur 46 du type PC qui gère automatiquement, à la fois le pilotage des divers sous-ensembles (18, 26, 38) de l'installation, le traitement et la représentation des données.

Pour chaque chaîne marquée, l'événement correspondant à son identification par spectrométrie de masse est caractérisé par la détection simultanée : 1) d'un marqueur caractérisant la famille de la chaîne, 2) d'un certain nombre d'autres marqueurs associés à chaque nucléotide de la chaîne. Ce nombre est directement relié à la quantité de nucléotides constituant la chaîne, donc à sa longueur (autant de marqueurs que de nucléotides par exemple).

Au bout d'un certain temps, de nombreuses chaînes sont passées une à une dans l'ensemble de détection. Une statistique est alors constituée afin d'établir des diagrammes tels que représentés sur la figure 3. Les diagrammes de la figure 3 correspondent à l'ADN hypothétique TGACTGCACGTA.

Le nombre d'événements associés à la présence d'un marqueur spécifique stable d'une famille (sur le didésoxy par exemple) est porté en ordonnée : isotope 1 pour les chaînes terminées par l'adénine (famille A) ; isotope 2 pour les chaînes terminées par la guanine (famille G) ; isotope 3 pour les chaînes terminées par la cytosine (famille C) et isotope 4 pour les chaînes terminées par la thymine (famille T). L'unité des ordonnées est arbitraire.

Le nombre de marqueurs stables associés aux nucléotides et détectés simultanément lors des événements est porté en abscisse. Ce nombre est directement relié (voire proportionnel) au nombre de nucléotides (n, n+1, n+2... avec n variant de 1 à 400 environ) constituant les chaînes détectées dans l'ensemble du système de détection.

Les diagrammes établis pour les quatre types de familles donnent alors les informations nécessaires pour déterminer l'ensemble de la séquence de l'ADN de départ.

A titre illustratif, on donne, ci-après, trois exemples de marquage des différentes chaînes de l'ADN hypothétique TGACTGCAACGTA.

Au cours des réactions de séquençage selon Sanger, tous les nucléotides libres présents (désoxy et didésoxy) sont marqués. Leur incorporation dans les bras synthétisés engendrent des chaînes de la forme:

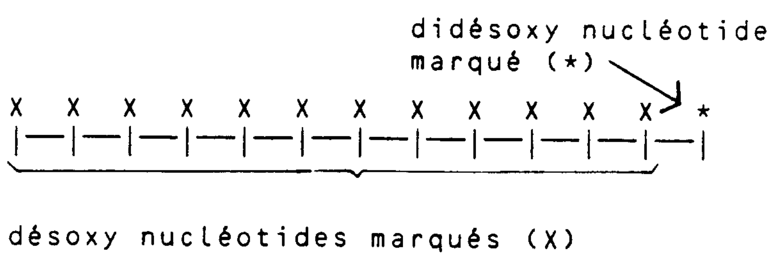

**EXEMPLE 1**

| désoxy nucléotides marqués marqués au $^{13}C$ : | didésoxy nucléotides marqués spécifiquement : |
|---|---|
| 2 - $^{13}C$ dATP<br>8 - $^{13}C$ dGTP<br>2 - $^{13}C$ dCTP<br>méthyl $^{13}C$ dTTP | 2,8 dichloro ddATP ($^{35}Cl$)<br>5,5 dibromo ddCTP ($^{79}Br$)<br>8,3′ difluoro ddGTP ($^{19}F$)<br>5,3′ diiodo ddTTP ($^{127}I$) |

Chaînes marquées

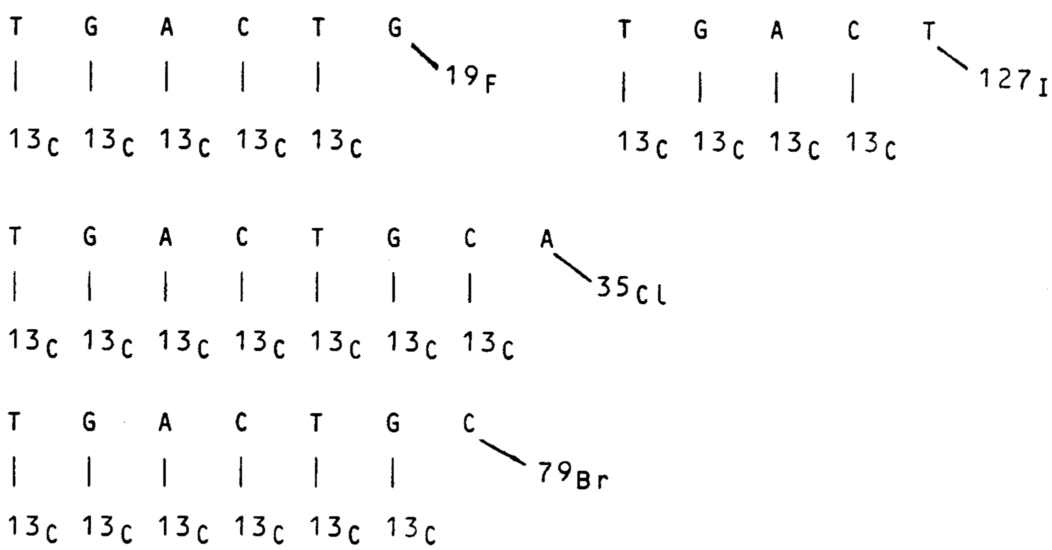


**EXEMPLE 2**

| désoxy nucléotides marqués au $^{32}S$ | didésoxy nucléotides marqués spécifiquement |
|---|---|
| α $^{32}S$ dATP<br>α $^{32}S$ dGTP<br>α $^{32}S$ dCTP<br>α $^{32}S$ dTTP | 1,3 $^{15}N$ ddATP<br>2,4 $^{13}C$ ddCTP<br>2,4 $^{18}O$ ddTTP<br>α,α $^{17}O$ ddGTP |

Exemples de chaînes marquées :

| | | | | | | |
|---|---|---|---|---|---|---|
| T | G | A | C | T | G | \ $^{17}O$ |
| $^{32}S$ | $^{32}S$ | $^{32}S$ | $^{32}S$ | $^{32}S$ | | |

| | | | | | |
|---|---|---|---|---|---|
| T | G | A | C | T | \ $^{18}O$ |
| $^{32}S$ | $^{32}S$ | $^{32}S$ | $^{32}S$ | | |

| | | | | | | | | |
|---|---|---|---|---|---|---|---|---|
| T | G | A | C | T | G | C | A | \ $^{15}N$ |
| $^{32}S$ | $^{32}S$ | $^{32}S$ | $^{32}S$ | $^{32}S$ | $^{32}S$ | $^{32}S$ | | |

| | | | | | | | |
|---|---|---|---|---|---|---|---|
| T | G | A | C | T | G | C | \ $^{13}C$ |
| $^{32}S$ | $^{32}S$ | $^{32}S$ | $^{32}S$ | $^{32}S$ | $^{32}S$ | | |

**EXEMPLE 3**

| désoxy nucléotides marqués au $^{79}Br$ | didésoxy nucléotides marqués spécifiquement : |
|---|---|
| 8 bromo dATP<br>8 bromo dGTP<br>5 bromo dCTP<br>5 bromo dTTP | α- $^{32}S$ ddATP<br>α- $^{33}S$ ddGTP<br>α- $^{34}S$ ddCTP<br>α- $^{36}S$ ddTTP |

Chaînes marquées

| | | | | | | |
|---|---|---|---|---|---|---|
| T | G | A | C | T | G | \ $^{33}S$ |
| $^{79}Br$ | $^{79}Br$ | $^{79}Br$ | $^{79}Br$ | $^{79}Br$ | | |

| | | | | | |
|---|---|---|---|---|---|
| T | G | A | C | T | \ $^{36}S$ |
| $^{79}Br$ | $^{79}Br$ | $^{79}Br$ | $^{79}Br$ | | |

| | | | | | | | | |
|---|---|---|---|---|---|---|---|---|
| T | G | A | C | T | G | C | A | \ $^{32}S$ |
| $^{79}Br$ | $^{79}Br$ | $^{79}Br$ | $^{79}Br$ | $^{79}Br$ | $^{79}Br$ | $^{79}Br$ | | |

| | | | | | | | |
|---|---|---|---|---|---|---|---|
| T | G | A | C | T | G | C | \ $^{34}S$ |
| $^{79}Br$ | $^{79}Br$ | $^{79}Br$ | $^{79}Br$ | $^{79}Br$ | $^{79}Br$ | | |

L'invention ayant été décrite en détail, il est évident que l'ARN ou d'autres types de molécules organiques complexes organiques peuvent être analysées grâce au procédé et à l'installation selon l'invention.

Par exemple en marquant chacun des acides aminés d'une protéine et plus spécifiquement certains d'entre eux, il est possible, grâce à l'invention de compter le nombre d'atomes marqueurs et d'en déduire le nombre d'acides aminés de la protéine ainsi que le nombre des acides aminés qui avaient été marqués spécifiquement. En renouvelant cette opération pour tous les types d'acides aminés, on peut déterminer la composition en acides aminés de la protéine.

De même façon, il est possible de déterminer la longueur des fragments de restriction d'ADN et d'ARN en comptant le nombre de marqueurs, celui-ci étant égal au nombre de nucléotides desdits fragments.

ANNEXE I

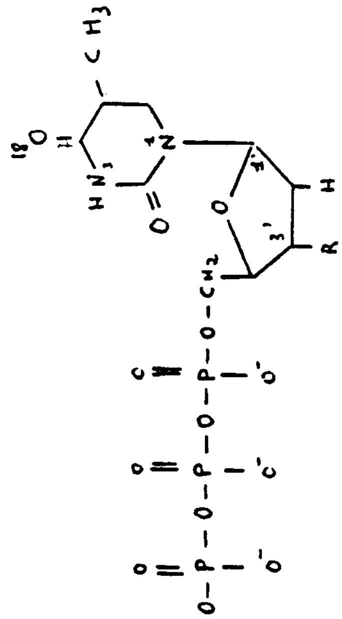

4. $^{18}O$ (d) dTTP

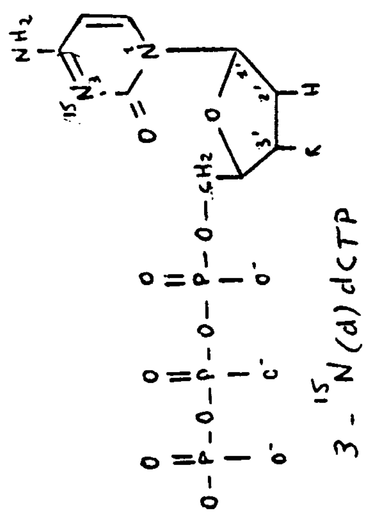

3. $^{15}N$ (d) dCTP

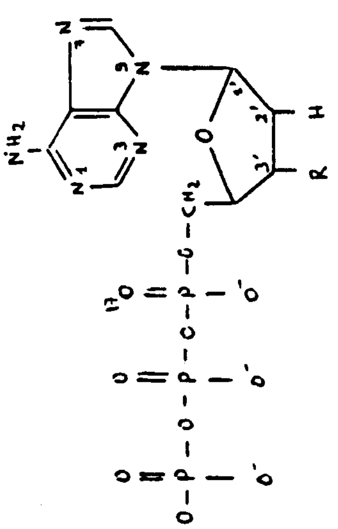

4. $^{17}O$ (d) dATP

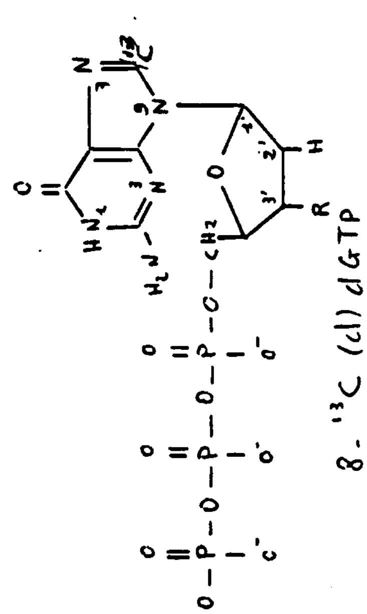

8. $^{13}C$ (d) dGTP

R = OH → dXTP
R = H → ddXTP

ANNEXE II

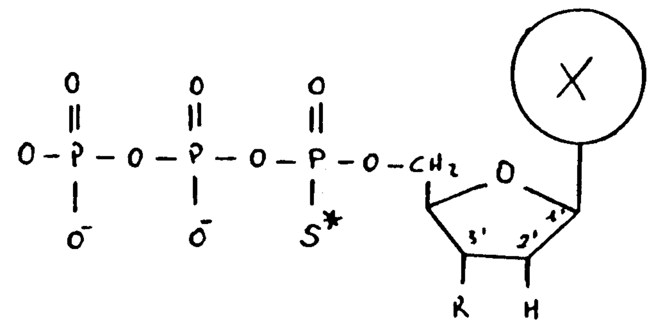

alpha - thio (d) d NTP

$S^* = {}^{32}S, {}^{33}S, {}^{34}S, {}^{36}S$

R = OH → d XTP

R = H → dd XTP

ANNEXE III

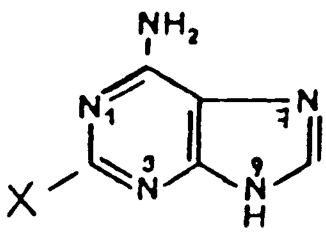

ADENINE

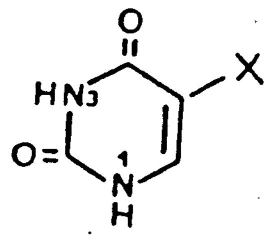

URACIL

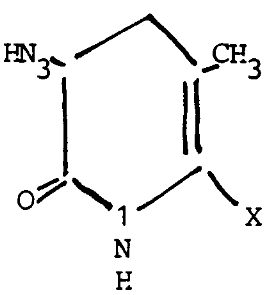

THYMINE

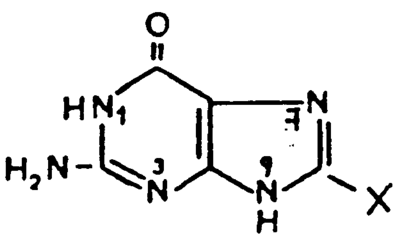

GUANINE

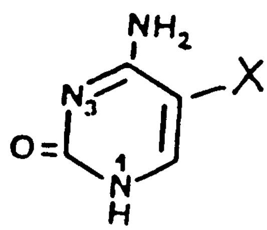

CYTOSINE

## Revendications

1. Procédé d'identification de la séquence des bases d'un acide nucléique, caractérisé en ce qu'il comprend les étapes suivantes :
   A) - production (2) de fragments de l'acide nucléique,
   B) - réactions chimiques spécifiques (4) des bases pour former des familles de chaînes de taille différente, chaque famille comportant à une extrémité une base spécifique,
   C) - marquage spécifique (6) desdites familles de chaînes et marquage de chaque nucléotide de chaque chaîne à l'aide d'isotopes stables ou de petites molécules stables directement greffables sur les nucléotides desdites chaînes.
   D) - introduction (8) une à une des chaînes marquées dans des moyens d'atomisation et d'ionisation (26, 28, 30, 32),
   E) - atomisation et ionisation (10) de chaque chaîne marquée à l'aide desdits moyens,

F) - sélection et détection (12) en temps réel des ions formés au fur et à mesure de leur formation, par spectrométrie de masse, et
G) -traitement (14) des signaux issus de la spectrométrie de masse, pour déterminer la longueur de chaque chaîne en comptant le nombre de nucléotides marqués de chaque ion détecté et pour identifier le marquage spécifique de chaque famille de chaînes.

**2.** Procédé selon la revendication 1, caractérisé en ce que le marquage est réalisé de façon intrinsèque avec au moins un isotope choisi parmi D, $^{13}C$, $^{15}N$, $^{17}O$, $^{18}O$, $^{32}S$, $^{33}S$, $^{34}S$, et $^{36}S$.

**3.** Procédé selon la revendication 1 ou 2, caractérisé en ce que le marquage est réalisé de façon extrinsèque avec au moins un isotope choisi parmi $^{19}F$, $^{35}Cl$, $^{37}Cl$, $^{79}Br$, $^{81}Br$ et $^{127}I$.

**4.** Procédé selon l'une quelconque des revendications 1 à 3, caractérisé en ce que l'acide nucléique est l'ADN.

**5.** Procédé selon l'une quelconque des revendications 1 à 4, caractérisé en ce que l'étape B) consiste à synthétiser une chaîne de nucléotides complémentaire d'un fragment dudit acide nucléique à partir d'un amorçeur jusqu'à ce qu'elle incorpore un didésoxynucléotide.

**6.** Procédé selon la revendication 5, caractérisé en ce que le marquage est effectué sur un élément choisi parmi la base, le sucre et le triphosphate du didésoxynucléotide et l'amorçeur.

**7.** Procédé selon la revendication 5 ou 6, caractérisé en ce que chaque nucléotide incorporé au cours de la synthèse de chaque chaîne est marqué.

**8.** Installation pour la mise en oeuvre du procédé selon l'une quelconque des revendications 1 à 7, caractérisée en ce qu'elle comporte :

- une source (18) de familles de chaînes de bases de longueur différente, chaque famille de bases étant spécifiquement marquée et les nucléotides de chaque chaîne étant marqués, ces marquages étant faits avec des isotopes stables ou de petites molécules stables directement greffées sur les nucléotides desdites chaînes,
- des moyens (22, 24) pour introduire une à une dans des moyens d'atomisation les chaînes marquées,
- des moyens (26, 28, 30) d'atomisation de chaque chaîne marquée,
- des moyens (32) d'ionisation des chaînes individualisées et atomisées,
- un spectromètre de masse (38) équipé de plusieurs détecteurs (42) pour sélectionner et détecter les ions formés au fur et à mesure de leur formation, et
- des moyens (46) de traitement des signaux fournis par les détecteurs (42), pour déterminer la longueur de chaque chaîne en comptant le nombre de nucléotides marqués de chaque ion détecté et pour identifier le marquage spécifique de chaque famille de chaînes.

**9.** Installation selon la revendication 8, caractérisée en ce que les moyens d'ionisation comportent une torche à plasma (32).

**10.** Procédé d'identification des motifs d'une molécule organique complexe à structure répétitive ou d'un fragment de cette molécule, comportant les étapes suivantes :

- marquage spécifique (6) desdits motifs par des isotopes stables ou petites molécules stables directement greffables sur lesdits motifs,
- introduction (8) un à un des motifs marqués dans des moyens d'atomisation et d'ionisation (26, 28, 30, 32),
- atomisation et ionisation (10) de chaque motif marqué à l'aide desdits moyens,
- détection (12) en temps réel des ions formés au fur et à mesure de leur formation par spectrométrie de masse, et
- traitement (14) des signaux issus de la spectrométrie de masse pour déterminer la longueur de la molécule complexe ou du fragment de cette molécule en comptant le nombre de motifs marqués et pour identifier leur marquage spécifique.

## Claims

**1.** Method for identifying the sequence of the bases of a nucleic acid, characterized in that it essentially comprises the following stages:

A) production (2) of fragments of the nucleic acid,

B) specific chemical reactions (4) of bases so as to form chains of families of different sizes, each family comprising at each extremity a specific base,

C) specific labelling (6) of said families of chains and labelling of each nucleotide of each chain with stable isotopes stable small molecules directly graftable onto the nucleotides of said chains,

D) introducing (8) one at a time labelled chains into atomizing and ionizing means (26, 28, 30, 32),

E) atomizing and ionizing (10) of each labelled chain by means of said means,

F) real time selecting and detecting (12) of ions formed as they form, by mass spectrometry and,

G) processing (14) signals derived from the mass spectrometer so as to determine the length of each chain by counting the number of labelled nucleotides of each ion detected and so as to identify the specific labelling of each family of chains.

**2.** Method according to claim 1, characterized in that labelling is intrinsically carried out with at least one isotope selected from D, $^{13}C$, $^{15}N$, $^{17}O$, $^{18}O$, $^{32}S$, $^{33}S$, $^{34}S$ and $^{36}S$.

**3.** Method according to claim 1 or 2, characterized in that labelling is extrinsically carried out with at least one isotope selected from $^{19}F$, $^{35}Cl$, $^{37}Cl$, $^{79}Br$, $^{81}Br$ and $^{127}I$.

**4.** Method according to any one of the claims 1 to 3, characterized in that the nucleic acid is DNA.

**5.** Method according to any one of the claims 1 to 4, characterized in that stage B) consists of synthesizing a complementary chain of nucleotides with a fragment of said nucleic acid from a primer until it incorporates a dideoxynucleotide.

**6.** Method according to claim 5, characterized in that labelling is carried out on an element selected from the base, sugar and triphosphate of the dideoxynucleotide and the primer.

**7.** Method according to claim 5 or 6, characterized in that each nucleotide incorporated during the synthesis of each chain is labelled.

**8.** Installation for performing the method according to any one of the claims 1 to 7, characterized in that it comprises:

- a source (18) of families of chains of bases of different length, each family of bases being specifically labelled and the nucleotides of each chain being labelled, these labellings being performed with stable isotopes or stable small molecules directly grafted onto the nucleotides of said chains,
- means (22, 24) for introducing the labelled chains one at a time into the atomization means,
- means (26, 28, 30) for atomizing each labelled chain,
- means (32) for ionizing individualized and atomized chains,
- a mass spectrometer (38) equipped with several detectors (42) for selecting and detecting the ions formed as they form and
- means (46) for processing signals supplied by the detectors (42) so as to determine the length of each chain by counting the number of labelled nucleotides of each ion detected and so as to identify the specific labelling of each family of chains.

**9.** Installation according to claim 8, characterized in that the ionization means comprises a plasma torch (32).

**10.** Method for identifying the units of a complex organic molecule with a repetitive structure or a fragment of this molecule, comprising the following stages:

- specific labelling (6) of said units by stable isotopes or stable small molecules directly graftable onto said units,
- introducing (18) one at a time the labelled units into the atomization and ionization means (26, 28, 30, 32),

- atomizing and ionizing (10) of each labelled unit using said means,
- detecting (12) in real time the ions formed, as they form, by mass spectrometry, and
- processing (14) signals derived from the mass spectrometer so as to determine the length of the complex molecule or fragment thereof by counting the number of labelled units and so as to identify their specific labelling.

**Patentansprüche**

1. Verfahren zur Identifikation der Sequenz von Nukleinsäurebasen, dadurch gekennzeichnet, daß es die folgenden Abschnitte umfaßt:
   A) - Herstellung (2) von Nukleinsäurefragmenten,
   B) - spezifische chemische Reaktionen (4) von Basen, um Familien von Ketten verschiedener Größe zu bilden, wobei jede Familie an einem Ende eine spezifische Base umfaßt,
   C) - spezifische Markierung (6) der besagten Familien von Ketten und Markierung jedes Nukleotids jeder Kette mithilfe von stabilen Isotopen oder kleinen, direkt auf die Nukleotide der besagten Ketten implantierten stabilen Molekülen,
   D) - Einführung (8) der markierten Ketten nacheinander in die Geräte zur Zerstäubung und Ionisierung (26, 28, 30, 32),
   E) - Zerstäubung und Ionisierung (10) jeder markierten Kette mithilfe der besagten Geräte,
   F) - Selektion und Detektion (12) der gebildeten Ionen in Echtzeit je nach ihrer Bildung durch Massenspektrometrie, und
   G) - Verarbeitung (14) der vom Massenspektrometer ausgegebenen Signale, um die Länge jeder Kette zu bestimmen, indem man die Anzahl der markierten Nukleotide jedes detektierten Ions zählt und um die spezifische Markierung jeder Familie der Ketten zu identifizieren.

2. Verfahren gemäß Anspruch 1, dadurch gekennzeichnet, daß die Markierung intrinsisch mit mindestens einem Isotop ausgeführt wird, das aus D, $^{13}C$, $^{15}N$, $^{17}O$, $^{18}O$, $^{32}S$, $^{33}S$, $^{34}S$ und $^{36}S$ ausgewählt wurde.

3. Verfahren gemäß Anspruch 1 oder 2, dadurch gekennzeichnet, daß die Markierung äußerlich mit mindestens einem Isotop ausgeführt wird, das aus $^{19}F$, $^{35}Cl$, $^{37}Cl$, $^{79}Br$, $^{81}Br$ und $^{127}I$ ausgewählt wurde.

4. Verfahren gemäß einem der Ansprüche 1 bis 3, dadurch gekennzeichnet, daß die Nukleinsäure DNS ist.

5. Verfahren gemäß einem der Ansprüche 1 bis 4, dadurch gekennzeichnet, daß der Abschnitt B) daraus besteht, eine zum Fragment der besagten Nukleinsäure komplementäre Nukeotidkette zu synthetisieren, ausgehend von einem Initiator bis sie ein Bidesoxynukleotid eingebaut hat.

6. Verfahren gemäß Anspruch 5, dadurch gekennzeichnet, daß die Markierung auf einem Element ausgeführt wird, das aus der Base, dem Zucker und dem Bidesoxynukleotidtriphophat und dem Initiator ausgewählt wird.

7. Verfahren gemäß Anspruch 5 oder 6, dadurch gekennzeichnet, daß jedes im Laufe der Synthese von jeder Kette eingebaute Nukleotid markiert ist.

8. Ausrüstung für den Einsatz des Verfahrens gemäß einem der Ansprüche 1 bis 7, dadurch gekennzeichnet, daß sie
   - eine Quelle (18) von Familien von Basenketten verschiedener Länge umfaßt, wobei jede Basenfamilie spezifisch markiert ist und die Nukleotide jeder Kette markiert sind, wobei diese Markierungen mit stabilen Isotopen oder kleinen stabilen, direkt auf die Nukleotide der besagten Ketten implantierten Molekülen gemacht werden,
   - Geräte (22, 24) umfaßt, um die markierten Ketten eine nach der anderen in die Geräte zur Zerstäubung einzuführen,
   - Geräte (26, 28, 30) zur Zerstäubung jeder markierten Kette umfaßt,
   - Geräte (32) zur Ionisierung der abgetrennten und zerstäubten Ketten umfaßt,
   - ein Massenspektrometer (38) umfaßt, das mit mehreren Detektoren (42) ausgerüstet ist, um die gebildeten Ionen je nach ihrer Bildung zu detektieren, und

- Geräte (46) zur Verarbeitung der von den Detektoren (42) gelieferten Signale, um die Länge jeder Kette zu bestimmen, indem man die Anzahl der markierten Nukleotide jedes detektierten Ions zählt und um die spezifische Markierung jeder Familie der Ketten zu identifizieren.

**9.** Ausrüstung gemäß Anspruch 8, dadurch gekennzeichnet, daß die Geräte zur Ionisierung einen Plasmabrenner (32) umfassen.

**10.** Verfahren zur Identifikation der Grundeinheit eines komplexen organischen Moleküls mit sich wiederholender Struktur oder eines Fragments dieses Moleküls, welches die folgenden Abschnitte umfaßt:
- spezifische Markierung (6) der besagten Grundeinheit durch stabile Isotopen oder kleine stabile, direkt in die besagten Grundeinheiten implantierten Moleküle,
- Einführung (8) der markierten Grundeinheiten eine nach der anderen in die Geräte zur Zerstäubung und Ionisierung (26, 28, 30, 32),
- Zerstäubung und Ionisierung (10) jeder markierten Grundeinheit mithilfe der besagten Geräte,
- Detektion (12) der gebildeten Ionen in Echtzeit je nach ihrer Bildung durch Massenspektrometrie, und
- Verarbeitung (14) der vom Massenspektrometer ausgegebenen Signale, um die Länge der komplexen Moleküle oder des Fragments dieser Moleküle zu bestimmen, indem die Anzahl der markierten Grundeinheiten gezählt wird und um ihre spezifische Markierung zu identifizieren.

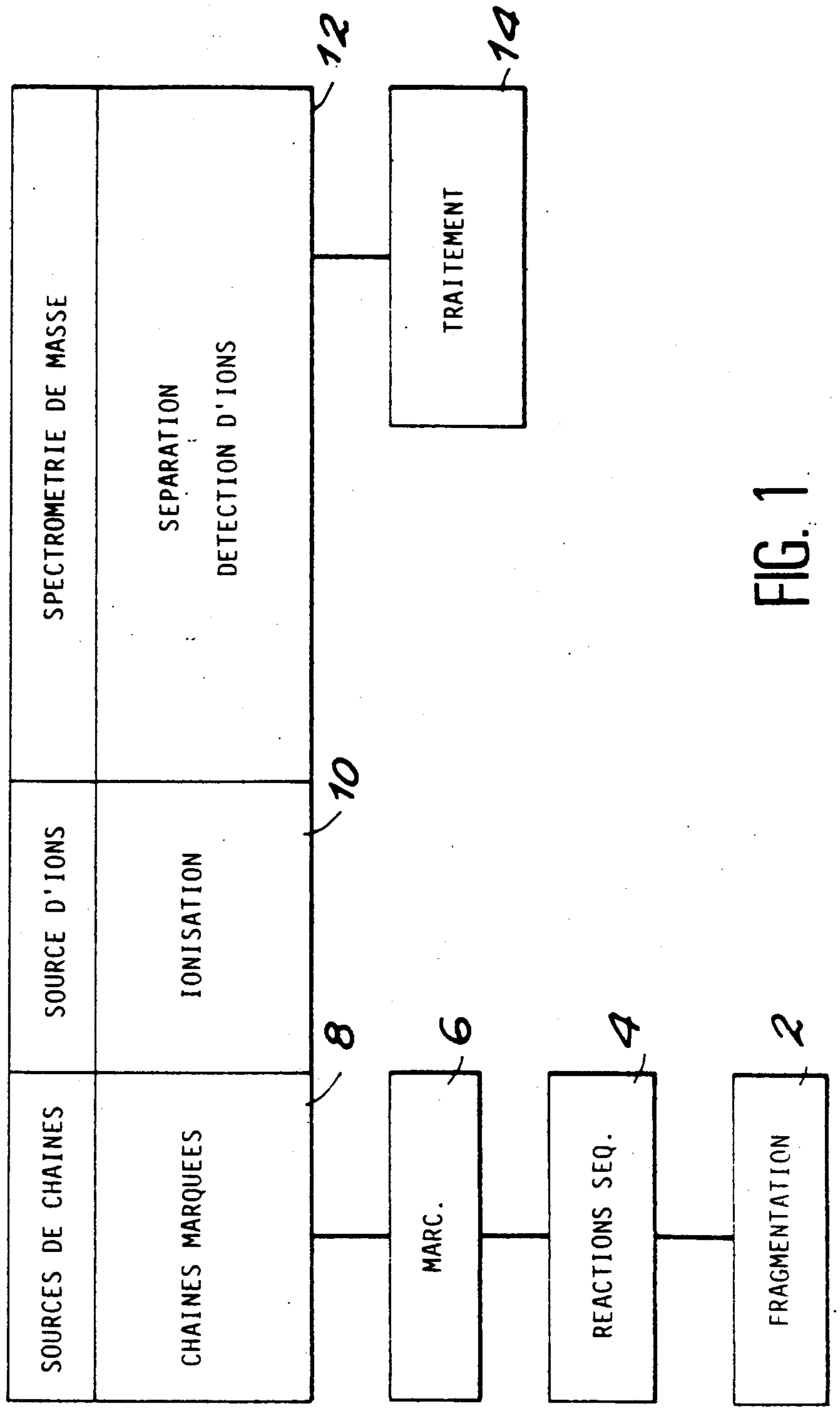
SPECTROMETRIE DE MASSE
SEPARATION
DETECTION D'IONS
12
TRAITEMENT
14
SOURCE D'IONS
IONISATION
10
SOURCES DE CHAINES
CHAINES MARQUEES
8
MARC.
6
REACTIONS SEQ.
4
FRAGMENTATION
2

FIG. 1

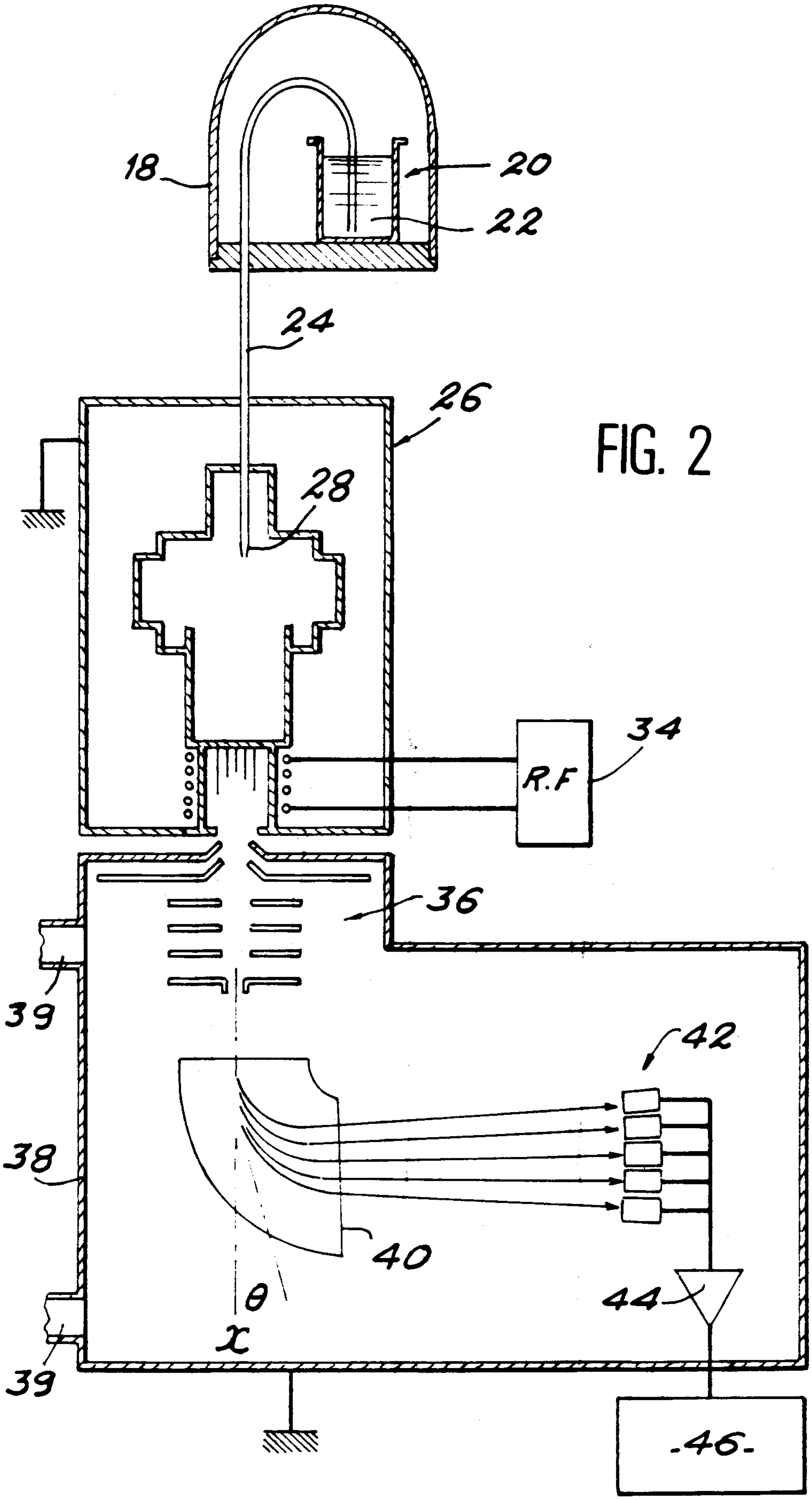


FIG. 2

FIG. 3

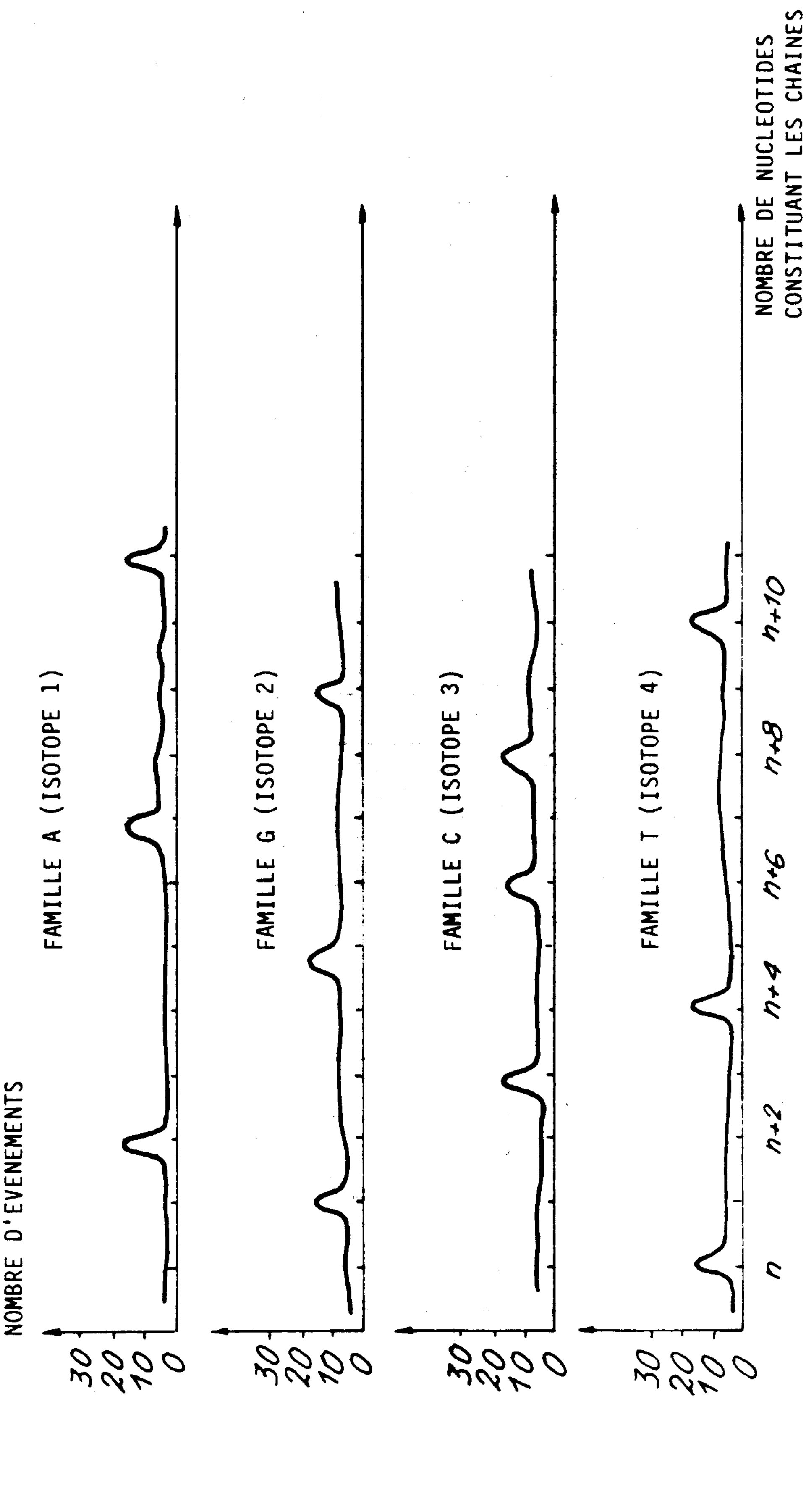


RESULTAT IDEALISE DE LA STATISTIQUE OBTENUE APRES LA DETECTION DE NOMBREUX EVENEMENTS CARACTERISANT LA DETECTION DES CHAINES